# FASCICULE DE BREVET EUROPEEN

(11) **EP 0 636 692 B1**
(45) Date de publication et mention de la délivrance du brevet: **09.03.2005**
(21) Numéro de dépôt: 94401741.7
(22) Date de dépôt: 28.07.1994
(51) Int. Cl.: C12N 1/04, A21D 8/04, A23K 1/00, C12G 1/022

(54) **Biomasse stable à base de cellules de levure et de bactéries lactiques et procédé de préparation**
Stabile Biomasse auf Basis von Hefezellen und Milchsäurebakterien und Verfahren zur Herstellung
Stable biomass based on yeast cells and lactic acid bacteria and process for its preparation

(30) Priorité: 29.07.1993 FR 9309373
(43) Date de publication de la demande: 01.02.1995
(73) Titulaire: LESAFFRE et Cie, F-75001 Paris (FR)
(72) Inventeur: Bonnardel, Pascal, F-59700 Marcq-en-Baroeul (FR); Taillade, Patrick, F-59420 Mouvaux (FR); Roques, Christian, F-59910 Bondues (FR); Thonart, Philippe, B-5081 La Bruyère (BE); Zgoulli, Slim, B-4031 Angleur (BE); Roblain, Dominique, B-1050 Bruxelles (BE)
(74) Mandataire: Koch, Gustave

(56) Documents cités:
- EP-A- 0 339 750
- CH-A- 240 611
- DE-C- 654 638
- FR-A- 912 852
- GB-A- 576 953
- US-A- 3 466 174
- PATENT ABSTRACTS OF JAPAN vol. 8, no. 41 (C-211) (1478) 22 Février 1984 & JP-A-58 201 960 (SHIYOUWA SANGYO K.K.) 25 Novembre 1983
- CHEMICAL ABSTRACTS, vol. 114, no. 25, 24 Juin 1991, Columbus, Ohio, US; abstract no. 246177a, page 658 ; & JP-A-330 668 (KANEGAFUCHI CHEMICAL INDUSTRY CO., LTD.) 8 Février 1991

## Description

L'invention a pour objet une biomasse stable à base de cellules de levure et de bactéries lactiques.

Elle vise également le procédé de préparation de cette biomasse.

Selon l'invention, on associe ensemble avec un minimum de matières étrangères et sous forme sèche des cellules de levures et de bactéries lactiques de façon à leur assurer une grande survie lors du procédé de fabrication ainsi que lors de la conservation et de l'utilisation dans les applications alimentaires pour lesquelles elles sont destinées.

Les pratiques traditionnelles d'ajout de microorganismes dans la fabrication de produits alimentaires ont été étudiées au fil du temps et améliorées avec l'apparition de microorganismes sélectionnés et cultivés à l'état pur afin d'ensemencer massivement les préparations alimentaires.

L'objectif est alors de produire des aliments aux qualités organoleptiques et nutritionnelles constantes. Ces pratiques concernent principalement les secteurs de la laiterie, salaisonnerie, vinification et panification.

Les levures et bactéries sont également utilisées sous forme active (c'est-à-dire sous forme de cellules vivantes) en alimentation du bétail ; les biomasses correspondantes sont appelées probiotiques.

L'utilisateur rencontre dans la pratique :
- soit des cultures pures d'espèces connues de bactéries ou de levures ; chaque souche sélectionnée est cultivée de façon intensive et pure par des procédés adaptés et optimisés qui permettent d'atteindre après récolte des concentrations cellulaires très élevées au gramme ou au millilitre de produit, pouvant atteindre 10¹⁰ germes par gramme pour les levures et 10¹² germes par gramme pour les bactéries,
- soit des associations de différents genres ou espèces dont la composition exacte est indéterminée, issues de propagations traditionnelles dites "naturelles ou spontanées" à partir d'aliments donnant des levains peu concentrés en microorganismes, dans lesquels le substrat de fermentation est pondéralement très largement prépondérant.

Ces préparations d'une seule ou plusieurs souches sont peu stables, que ce soit à température ambiante ou bien au froid à 4°C. Leur conservation est donc très courte. Seules font exception les levures de panification qui peuvent se conserver jusqu'à 2 mois à 4°C.

Pour permettre une conservation supérieure à un mois de ces préparations constituées des cellules vivantes de bactéries et/ou de levures, le séchage à plus de 90% de matières sèches est généralement employé.

Le séchage des levures de panification et des levures oenologiques est une technique bien connue. On cultive la souche sous forme d'une biomasse pure, la levure est récoltée, mélangée avec des auxiliaires technologiques à raison de quelques pour cent de la matière sèche mise en oeuvre comme notamment des monostéarates de sorbitan, extrudée et séchée par différentes techniques: la fluidisation, le séchage sur bande en tunnel, le séchage en tambour rotatif. Ces différentes techniques sont notamment décrites dans des manuels de base comme YEAST TECHNOLOGY, REED AND NAGODAWITHANA, second edition, an AVI book, VAN NOSTRAND REINHOLD. La fluidisation est aujourd'hui la technique la plus généralement employée. De manière générale, l'atomisation est une technique qui donne de mauvais résultats pour le séchage des biomasses vivantes. La lyophilisation est une technique qui ne convient pas pour le séchage des levures.

Le séchage des bactéries lactiques est également une technique bien connue, bien que plus délicate. Elle est dans la pratique toujours pratiquée par lyophilisation en présence d'une quantité importante d'agents de support ou de dessiccation, qui ont un rôle crucial. En effet, l'article de Lou C Lievense et al - INACTIVATION OF LACTOBACILLUS PLANTARUM DURING DRYING, Bioseparation I, 161-170, 1990, montre que l'inactivation des bactéries lactiques pendant le séchage est due à deux mécanismes : l'inactivation thermique et surtout l'inactivation due à la dessiccation elle-même.

Le choix de la nature et de la quantité du support protégeant les bactéries lactiques des pertes d'activités dues à la dessiccation est donc très important. Ceci est par exemple exprimé dans le brevet US 4956295 qui montre que de nombreuses substances desséchantes sont utilisées lors du séchage et ensuite en mélange avec les bactéries lactiques pour les stabiliser.

L'état de la technique montre que les conditions optimales pour sécher des levures et des bactéries lactiques sont fort différentes. Ceci résulte notamment du fait déjà indiqué que la seule technique retenue industriellement pour le séchage des bactéries lactiques est la lyophilisation, alors que cette technique conduit à des hauts taux d'inactivation des levures. Dans la pratique, les levures et les bactéries sont donc toujours séchées séparément. La seule exception consiste en des séchages de levains cultivés sur farines ou autres substrats, suite à des ensemencements maîtrisés ou non de bactéries et de levures. Ces levains contiennent en général de très faibles teneur en cellules revivifiables. Un exemple de tels levains est celui décrit dans le brevet européen 0339750 où un ensemencement connu de souches de levure et de bactéries est cultivé sur farine; puis la culture est déshydratée par mélange avec de la farine sèche, le séchage du mélange étant terminé dans un courant d'air chaud.

Selon l'invention, on associe des bactéries à de la levure avec un ajout très faible d'auxiliaires technologiques, en faisant jouer à la biomasse de cellules de levures vivantes un rôle protecteur vis-à-vis des bactéries durant la dessiccation.

La présente invention est intéressante pour tout domaine d'application dans lequel on a besoin de levures et de bactéries associées. Elle présente pour avantage d'une part d'obtenir des produits homogènes composés de particules sèches associant levures et bactéries et d'autre part de ne pas apporter, lors de l'utilisation de ces particules, de support étranger. Elle remédie notamment au problème posé par le mélange de particules sèches hétérogènes contenant d'une part des levures et d'autre part des bactéries lactiques sur support. Elle permet de supprimer l'emploi de tout support venant dluer de manière significative la biomasse de cellules vivantes.

La biomasse conforme à l'invention comprend sous forme de petites particules substantiellement régulières et identiques d'une teneur en matière sèche supérieure à 92%, de préférence à 93%, d'une part au moins 1.10⁹ cellules revivifiables de bactéries lactiques, de préféence au moins 3.10⁹ et plus préférentiellement encore au moins 1.10¹⁰ cellules revivifiables de bactéries lactiques par gramme et, d'autre part, au moins 5.10⁹, de préférence au moins 1.10¹⁰ cellules revivifiables de levures par gramme, lesdites particules sèches comportant moins de 5%, de préférence moins de 3%, de support étranger essentiellement composé des auxiliaires technologiques classiques protégeant les cellules vivantes lors du séchage.

Cette biomasse sera composée:
- d'une part, d'une ou plusieurs souches de bactéries lactiques, c'est-à-dire des bactéries appartenant de préférence aux genres Lactobacillus ou Streptococcus ou Leuconostoc et de préférence aux espèces Lactobacillus brevis, Lactobacillus plantarum, Lactobacillus casei, Lactobacillus delbruekii, Lactobacillus San Francisco, Streptococcus faecium, Leuconostoc oenos,
- et, d'autre part, d'une ou plusieurs souches de levures, de préférence appartenant à la famille des Saccharomycetaceae, de préférence au genre Saccharomyces et encore plus préféreniellement à l'espèce Saccharomyces cerevisiae, la préférence allant pour l'utilisation en panification à une variété ne fermentant pas le maltose, comme le Saccharomyces cerevisiae chevalieri.

La biomasse associant des souches de levures et de bactéries, après culture industrielle et récolte, au sein de petites particules sèches substantiellement rfégulières et identiques, ayant la forme de granules ou de sphérules, à au moins 92% de matières sèches contenant au moins 95% de biomasse cellulaire (cellules de levures et de bactéries sur matière sèche totale) pourra être préparée selon l'invention par l'un des deux procédés suivants qui utilisent la levure comme agent protecteur des bactéries lactiques lors de la dessiccation.

Ainsi, conformément à l'invention,
- on mélange une crème de bactéries avec de la levure pressée ayant des matières sèches comprises entre environ 30 et 33% dans un malaxeur,
- on ajoute éventuellement à ce mélange pâteux des auxiliaires technologiques protégeant la biomasse lors du séchage comme par exemple le monostéarate de sorbian associé ou non avec de la carboxyméthylcellulose ou tout émulsifiant ou épaississant ayant les mêmes propriétés,
- on extrude cette biomasse en vermicelles de diamètre inférieur à 3 mm et de préférence inférieur à 1 mm,
- on sèche les vermicelles dans un courant d'air chaud comme par exemple le séchage par fluidisation jusqu'à au moins 92 % de matières sèches et de préférence à au moins 93 % de matière sèche.

Egalement, conformément à l'invention, on opère de la manière suivante :
- on sèche séparément la levure de manière à obtenir de fins granules de diamètre inférieur à 1 mm, de préférence à au moins 95 % de matières sèches en présence des auxiliaires technologiques de séchage utilisés lors de la production des levures sèches instantanées comme le monostéarate de sorbitan par exemple,
- on pulvérise sur lesdits granules de levure en mouvement dans un mélangeur ou en fluidisation une crème de bactéries lactiques à au moins 14 % de matières sèches contenant éventuellement des auxiliaires technologiques les protégeant lors de la dessiccation comme par exemple du glycérol ou tout produit ayant un effet protecteur équivalent,
- on effectue éventuellement un séchage complémentaire dans un courant d'air chaud comme un séchage par fluidisation de manière à amener les granules à au moins 92 % de matières sèches et de préférence à au moins 93 % de matières sèches.

Le susdit premier procédé conforme à l'invention permet d'obtenir des granules ou sphérules secs homogènes. Il permet notamment d'obtenir par des techniques de séchage comme le séchage en lit fluidisé à forte hauteur de couche ou le séchage en séchoir rotatif ou tambour des granules ou des sphérules lisses et non poreux donnant des probiotiques ayant des propriétés nouvelles.

Le susdit second procédé selon l'invention qui donne des granules substantiellement identiques constitués de cellules de levures enrobés à leur périphérie par les cellules de bactéries lactiques sera préféré pour l'obtention de ferments panaires ou viniques.

L'invention pourra être encore mieux comprise à l'aide des exemples ci-après non limitatifs et relatifs à des modes de réalisation avantageux ; elle permet d'obtenir des produits très concentrés en levures et bactéries vivantes avec une charge protectrice étrangère très réduite, présentant une longue conservation, supérieure à 3 mois à 20°C et 6 mois à moins de 10°C, de préférence 12 mois à moins de 10°C (environ 4°C).

### EXEMPLE 1: PROCEDE DE FABRICATION D'UN FERMENT POUR LA PANIFICATION.

Les souches travaillées sont :
- d'une part une levure : Saccharomyces cerevisiae variété chevalieri NCYC 935,
- d'autre part deux bactéries isolées de levains naturels de panification française sur froment :
   Lactobacillus casei
   Lactobacillus brevis.

### Etape 1 : Propagation des souches

Parallèlement et sur des équipements différents, les souches de la levure et bactéries sont cultivées et produites en quantité importante selon les procédés suivants :
1. La levure est produite selon les méthodes habituelles de propagation des levures de panification bien connues par ailleurs, telles que décrites dans YEAST TECHNOLOGY, Second edition, REED AND NAGODAWITHANA, AVI BOOK (ISBN 0-442-51892-8) 1991.
   Classiquement le moût de fermentation est séparé de la crème de levure et filtré jusqu'à l'obtention d'un produit pâteux à environ 33 % de matière sèche qui sera stocké à 4 °C dans l'attente de l'étape 2 suivante.
2. Les 2 souches de bactéries sont propagées séparément en fermenteurs selon des procédés classiques bien connus par ailleurs employant un milieu de type MRS pour les précultures et cultures finales, tels que décrits dans "BERGEY'S Manual of systematic bacteriology - volume 2", SNEATH, P.H.A. ; MAIR, N.S. ; SHARPE, M.E. and HOLT, J.G. (Eds), WILLIAMS AND WILKINS (Publ.), 1986, Baltimore. On obtient en fin de fermentation des concentrations cellulaires d'environ 10¹⁰ cfu/ml (cfu = nombre de cellules viables par unité en l'occurence par ml) ; Chaque moût est ensuite centrifugé pour fournir deux crèmes de bactéries possédant les caractéristiques suivantes :

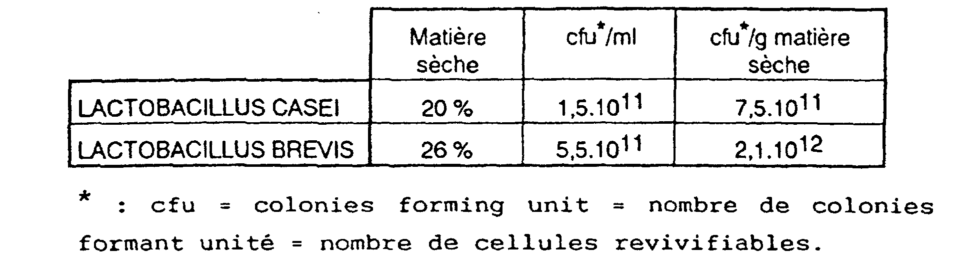

Les deux crèmes sont conservées au froid à 4 °C dans l'attente de l'étape 2 suivante :

### Etape 2 : coséchage des levures et bactéries ensemble

Les deux crèmes de bactéries issues de l'étape (1) sont ajoutées à la levure obtenue à l'issue de la même étape dans les proportions pondérales suivantes :

| | |
|---|---|
| Levure | 99,34 % |
| Lactobacillus casei | 0,58 % |
| Lactobacillus brevis | 0,08 % |

Ceci correspond à environ 2,6 . 10⁹ cellules de Lactobacillus casei par gramme de matière sèche du mélange et environ 1,3 10⁹ cellules de Lactobacillus brevis par gramme de matière sèche, soit au total 3,9.10⁹ cfu/g de matière sèche. Les proportions souche de levure et souches de bactéries lactiques homofermentaire (c'est-à-dire produisant uniquement de l'acide lactique) et hétérofermentaire (c'est-à-dire produisant notamment aussi de l'acide acétique) sont celles correspondant à la fabrication d'un ferment panaire. Des proportions beaucoup plus importantes de biomasse de bactéries lactiques par rapport aux levures sont possibles, étant entendu que de préférence la levure représentera au moins 50 % et encore de préférence au moins 80 % de la biomasse soumise au séchage.

Des auxiliaires technologiques de séchage habituellement utilisés pour le séchage de la levure de boulangerie sont employés comme par exemple une émulsion à base de monostearate de sorbitan apporté à 1,5 % par rapport à la matière sèche de la biomasse et de carboxy-méthyl-cellulose apporté à 1 % par rapport à la matière sèche de la biomasse

Après le mélange intime de ces 3 microorganismes, l'ensemble possédant une matière sèche totale d'environ 33 % est extrudé au travers d'une filière et séché de manière ménageante dans un courant d'air déshydraté et chaud pour obtenir en final de fins vermicelles de diamètre d'environ 0,6 mm et de longueur d'environ 1 mm contenant plus de 95 % de matières sèches. Un séchage ménageant est défini comme un séchage durant moins d'une heure où la température de la biomasse ne dépasse pas 40°C et de préférence 35°C. A la fin du séchage, la biomasse sèche est immédiatement refroidie à moins de 20°C et mise sous atmosphère inerte.

Ce ferment ainsi réalisé possède toutes les propriétés d'utilisation instantanée de la levure sèche de boulangerie instantanée.

LES RESULTATS POUR LE FERMENT OBTENU OU FERMENT PANAIRE 1 sont les suivants :

| Teneur bactérienne initiale avant séchage cfu/g de matière sèche | Matière sèche du ferment 1 % | Teneur en cellules vivantes cfu/g matière sèche du ferment 1 | | Pertes bactériennes au séchage en % |
|---|---|---|---|---|
| | | Levures | Bactéries | |
| 3.9.10⁹ | 96 | 1,1.10¹⁰ | 2,6.10⁹ (dont 2/3 de L. casei et 1/3 de L. brevis) | 33 |

Le ferment est conditionné sous atmosphère inerte ou sous vide (moins de 1% d'oxygène résiduel) et présente une parfaite stabilité après 3 mois de conservation à température ambiante c'est-à-dire entre 20 et 23 °C et après 12 mois de conservation à 4 °C.

### EXEMPLE 2 : PROCEDE DE FABRICATION D'UN FERMENT POUR PANIFICATION

Les souches utilisées sont les mêmes que celles de l'exemple 1. De même l'étape 1 de propagation des souches est la même.

Les crèmes de bactéries ont les caractéristiques suivantes :

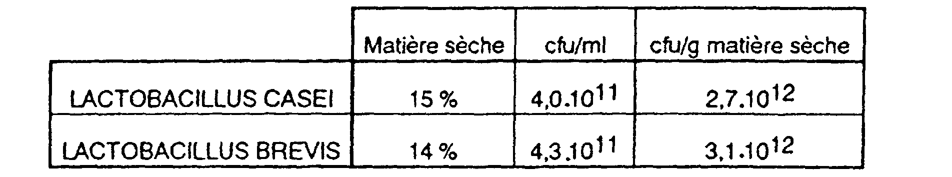

Les deux crèmes sont conservées au froid à + 4°C dans l'attente de l'étape suivante :

### Etape 2 : séchage des levures et des bactéries

Une émulsion apportant 1,5 % de monostearate de sorbitan par rapport à la matière sèche levure est mélangée à la levure Saccharomyces chevalieri à 33 % de matière sèche. Celle-ci est ensuite extrudée et séchée de manière ménageante dans un courant d'air sec et chaud. On obtient alors des vermicelles, de levure, de diamètre d'environ 0,3 à 0,6 mm, de longueur d'environ 1 mm et de matière sèche 95 % contenant environ 1,2.10¹⁰ cfu par gramme de matière sèche.

Par ailleurs, on réalise le mélange dans les proportions suivantes pour obtenir une crème de faible viscosité facilement pompable à environ 20 % de matières sèches et contenant environ 3,5 . 10¹¹ cfu bactéries lactiques par ml :

| | |
|---|---|
| Crème de lactobacillus casei | 58 ml |
| Crème de lactobacillus brevis | 27 ml |
| Levure sèche Saccharomyces cerevisiae chevalieri | 8 g |
| Eau | q.s.p. 100 g |

La crème du mélange ci-dessus est pulvérisée à raison de 5 g pour 100 g de produit obtenu sur les particules de levure sèche instantanée Saccharomyces cerevisiae chevalieri mise en mouvement dans un mélangeur cylindrique rotatif muni de pales de brassage créant une fine pluie tombante de vermicelles (5 g de crème pour 95 g de levure sèche instantanée).

Le produit obtenu possède une matière sèche d'environ 91 % et contient environ 1,8.10¹⁰ bactéries revivifiables par gramme de matière sèche. On le sèche ensuite rapidement et de manière ménageante dans un courant d'air déshydraté et chaud en lit fluidisé pour l'amener à environ 95 % de matière sèche. On obtient alors des granules ayant environ 1,2.10¹⁰ cfu levures par gramme de matière sèche et environ 1,5 . 10¹⁰ cfu bactéries lactiques par gramme de matière sèche. Ce produit peut éventuellement être mélangé à raison de 20 % à de la levure sèche Saccharomyces cerevisiae chevalieri obtenue comme ci-dessus pour ajuster le nombre de lactobacilles à environ 3.10⁹ cfu/g de matière sèche dont environ 2/3 de Lactobacillus casei et environ 1/3 de Lactobacillus brevis pour 1,2.10¹⁰ cfu levures par gramme de matière sèche. Ce mélange éventuel 20/80 ne pose aucune difficulté toutes les particules ayant en fait même taille et même densité, il se fait sans aucune perte de viabilité.

LES RESULTATS POUR LE FERMENT PANAIRE 2 sont les suivants :

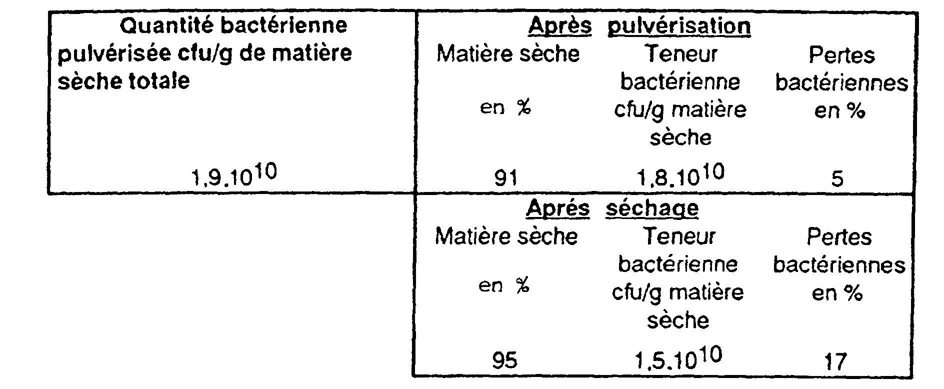

Le ferment est conditionné sous atmosphère inerte et présente une parfaite stabilité après 3 mois de conservation à température ambiante, c'est à dire entre 20 et 23°C et après au moins 6 mois de conservation à 4°C.

### EXEMPLE 3 : PROCEDE DE FABRICATION D'UN FERMENT POUR PANIFICATION

Les souches utilisées sont toujours les mêmes que celles de l'exemple 1. De même l'étape 1 de propagation reste inchangée, les crèmes de bactéries ont les caractéristiques suivantes :

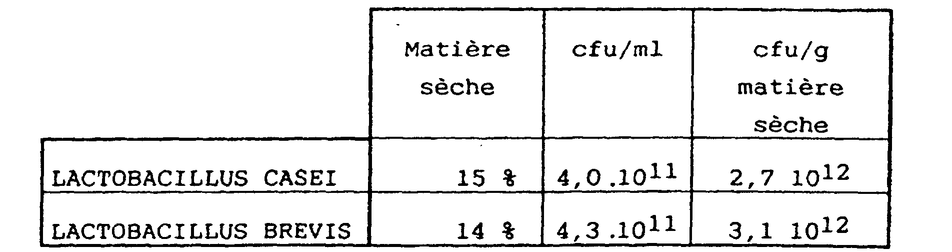

Les deux crèmes sont conservées au froid à 4°C dans l'attente de l'étape 2 suivante :

### Etape 2 : Séchage des levures et bactéries

Dans un premier temps comme cela a été décrit dans l'exemple 2, la levure Saccharomyces cerevisiae chevalieri est séchée sous forme de fins vermicelles de mêmes dimensions et de matière sèche finale 95 %. Elle est placée dans un mélangeur cylindrique rotatif muni de pales de brassage du même type que celui employé dans l'exemple 2.

Par la suite, on réalise le mélange dans les proportions pondérales suivantes :

| | |
|---|---|
| crème de Lactobacillus casei | 62 % |
| crème de Lactobacillus brevis | 28% |
| glycérol | 10 % |

Ce mélange pompable est maintenu à + 4 °C. Il est pulvérisé sur la levure sèche Saccharomyces cerevisiae chevalieri en mouvement à raison d'un peu plus de 1 %, c'est-à-dire de manière à apporter une teneur en cellules bactériennes d'au moins 3,9.10⁹ cfu/g de matière sèche cellulaire totale. Aussitôt le sprayage terminé, le ferment réalisé associant la levure Saccharomyces cerevisiae chevalieri aux 2 bactéries Lactobacillus casei et Lactobacillus brevis est conditionné sous gaz inerte et stocké à + 4 °C. Le produit est parfaitement stable pendant au moins 6 mois à une température de 4 °C.

LES RESULTATS POUR LE FERMENT PANAIRE 3 sont les suivants :

| Teneur bactérienne initiale avant séchage cfu/g de matière sèche | Matière sèche du ferment % | Teneur en cellules vivantes du ferment 3 cfu/g matière sèche | | Pertes bactériennes au séchage en % |
|---|---|---|---|---|
| | | Levures | Bactéries | |
| 3,9.10⁹ | 94 | 1,2.10¹⁰ | 3.10⁹ (dont 2/3 de L. casei et 1/3 de L. brevis) | 23 |

### EXEMPLE 4 : FABRICATION DE PAINS AU LEVAIN A PARTIR DES FERMENTS PRODUITS SELON LES EXEMPLES 1, 2 ET 3.

On réalise les 4 essais de panification suivants selon la formule et les procédés décrits ci-après.
essai 1 :Pain au levain fabriqué avec le ferment panaire 1 de l'exemple 1.
essai 2 :Pain au levain fabriqué avec le ferment panaire 2 de l'exemple 2 standardisé à environ 3.10⁹ cfu bactéries lactiques /g
essai 3 : Pain au levain fabriqué avec le ferment panaire 3 de l'exemple 3.
essai 4 : Témoin levain "naturel" ou spontané.

### 1 - FABRICATION DES LEVAINS

### 1.1 - Formules

### Levain témoin de l'essai 4.

### 1ère étape :

| | |
|---|---|
| Farine (18 °C) | 100 % |
| Eau (25°C) | 57 % |
| Levain chef | 50 % |
| Sel | 1,8 % |
| Fermentation | 8h à 24 °C |

### 2ème étape :

| | |
|---|---|
| Farine (18 °C) | 100 % |
| Eau (25 °C) | 57 % |
| Levain 1ère étape | 34 % |
| Sel | 1,8 % |
| Fermentation | 16 h à 21°C |

On obtient alors un levain tout point qui servira d'une part comme levain chef pour relancer un levain première étape et d'autre part à la fabrication de la pâte lors du pétrissage final. Au départ du cycle, on est parti d'un levain ensemencé par les bactéries et les levures présentes à titre de "contaminants naturels" dans les ingrédients (farine essentiellement) et l'air, qu'on a soigneusement sélectionné et rafraîchi régulièrement.

Tous les pourcentages sont calculés par rapport à 100 de farine.

### Levain témoin de l'essai 1, 2 et 3

| | |
|---|---|
| Farine (18 °C) | 100 % |
| Eau (25 °C) | 55 % |
| Ferment panaire 1 ou 2 ou 3 | 0,5 % |
| Sel | 1,8 % |
| Fermentation | 18h à 27°C. |

Caractéristiques de la farine utilisée pour les 4 essais :

| | |
|---|---|
| Type | 55 |
| Teneur en eau | 14,2 % |
| Protéine sur matière sèche | 14,3 % |
| Hagberg | 330 |
| P | 80 |
| G | 23,7 |
| P/L | 0,7 |
| W | 290 |
| Acide ascorbique | environ 10 ppm |
| alpha-amylases fongiques | 0 |

P, G, P/L, W sont les caractéristiques alvéographiques mesurées selon la méthode standard à l'alvéographe de Chopin.

### 1.2 - Schémas et procédés de fabrication

Le pétrissage lors de chaque étape du levain témoin ou bien pour les essais avec les ferments est réalisé sur pétrin type ARTOFEX 4 minutes à 40 plongées/minute suivi des fermentations indiquées dans les formules ci-dessus.

### 1.3 - Résultats

Les levains obtenus et avant utilisation dans la pâte finale présentent les caractéristiques suivantes :

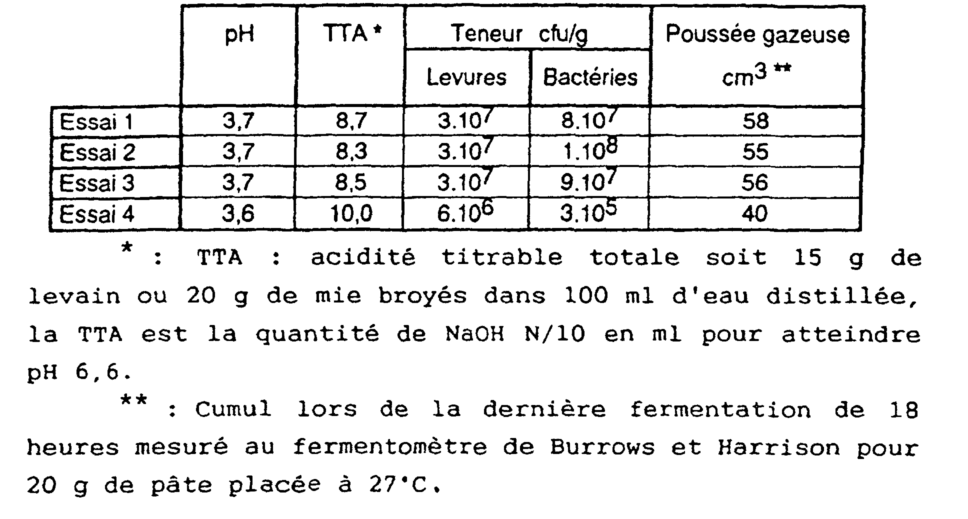

### 2 - FABRICATION DES PAINS

### 2.1 - Formules

| | |
|---|---|
| Farine (18°C) | 100 % |
| Eau (24°C) | 64 % |
| Levain des essais 1-2-3-4 | 30 % |
| Sel | 1,8 % |
| Levure de boulangerie "Hirondelle Bleue" ® | 0,2 % |
| Alpha-amylases (complexe fongique) : | 40 000 SKB/quintal de farine. |

Caractéristiques de la farine utilisée pour les 4 essais :

| | |
|---|---|
| Type | 55 |
| Teneur en eau | 15,3 % |
| Protéines sur matières sèches | 10,5 % |
| Hagberg | 320 |
| P | 72 |
| G | 20,8 |
| P/L | 0,8 |
| W | 210 |
| Acide ascorbique | 20 ppm |
| Alpha-amylases fongiques | 0 |

### 2.2 - Schémas et procédés de fabrication

- Mélange de tous les ingrédients dans un pétrin de type ARTOFEX à bras plongeants sauf le levain et la levure.
- Repos ou autolyse de 30 minutes.
- Ajout levain et levure.
- Pétrissage final sur pétrin ARTOFEX :
   . 1 minute à 40 plongées/minute.
   . 11 minutes à 60 plongées/minute.
- Température de la pâte 24°C.
- Première fermentation en masse 1h30 à 23°C.
- Division de la pâte en pâtons de 500 g suivie d'un repos de 26 minutes à 23°C.
- Façonnage manuel en bâtards courts.
- Deuxième fermentation : les pâtons sont déposés sur couches recouverts d'une couverture et placés dans un parisien (enceinte close) durant 4 heures à 23°C.
- Scarification et cuisson dans un four à sole avec au préalable injection de vapeur d'eau. Cuisson : 40 minutes à 220°C.

### 2.3 - Résultats

Les pains obtenus présentent les caractéristiques suivantes :

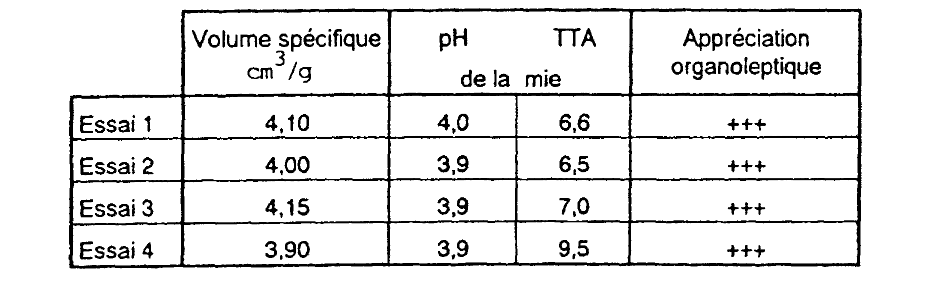

Les pains de différents essais sont tout à fait comparables entre eux ; ils présentent un aspect de la croûte et de la mie similaire, des qualités aromatiques, de goût, d'imbibition de la mie et de conservation similaires et caractéristiques de pains au levain.

L'utilisation d'un des 3 ferments panaires secs évite au boulanger d'avoir à démarrer un levain lorsqu'il ne dispose pas de levain spontané de bonne qualité. Elle lui évite l'obtention et l'entretien d'un levain tout point, opération longue, particulièrement délicate à maîtriser. Elle lui assure une qualité régulière et excellente de son pain au levain.

### EXEMPLE 5 : PROCEDE DE FABRICATION D'UN PROBIOTIOUE POUR ALIMENTATION ANIMALE

Les souches travaillées sont :
- d'une part, une souche de levure : **Saccharomyces cerevisiae,**
- d'autre part, une souche de bactérie : **Streptococcus faecium**.
qui ont été sélectionnées pour leurs propriétés probiotiques, c'est-à-dire leurs propriétés de survie dans le tractus digestif des animaux-cibles et leurs propriétés de régularisation, d'optimisation de la flore digestive desdits animaux-cibles. Est appelé probiotique, tout apport de cellules vivantes dans la ration des animaux ayant un effet positif sur l'utilisation de la ration par les animaux.

### Etape 1 : propagation des souches

Comme dans l'exemple 1, les deux souches sont cultivées séparément selon des pratiques usuelles de production de biomasse de levure d'une part, de bactéries lactiques d'autre part. On obtient d'une part une levure pressée à au moins 30 % de matière sèche et d'autre part, une crème bactérienne aux caractéristiques suivantes :

| Matière sèche (%) | Teneur en bactéries vivantes cfu/ml | Teneur en bactéries vivantes cfu/g matière sèche |
|---|---|---|
| 21,4 | 4.10¹¹ | 1,9.10¹² |

Les deux cultures sont conservées au froid à 4°C dans l'attente de la seconde étape suivante :

### Etape 2 : Coséchage des levures et bactéries ensemble de manière à obtenir des particules riches en organismes cellulaires revivifiables

On réalise comme dans l'exemple 1 le mélange intime dans un malaxeur de la crème de bactéries et la levure pressée dans une proportion de 100 g de crème de streptocoques pour 1 kg de levure à 31 % de matière sèche, auquel on ajoute les auxiliaires technologiques de séchage habituels (émulsion de monostéarate de sorbitan avec éventuellement de la carboxyméthylcellulose) quand le séchage a lieu par fluidisation.

Le mélange possède une matière sèche d'environ 30 %. Il est extrudé au travers d'une filière et séché à au moins 92 % de matières sèches dans un courant d'air déshydraté à 3 g d'eau maximum par kg d'air sec et chaud pendant plusieurs heures sur lit fluidisé à forte hauteur de couche pour obtenir en final de fins vermicelles de diamètre 0,6 mm à 1 mm et de longueur maximale d'environ 1 mm à 2 mm ou en séchoir rotatif utilisé en production de levure sèche de panification et donnant des petites sphérules, de manière à obtenir dans tous les cas des particules lisses non poreuses. Les temps de séchage durent plusieurs heures et sont suffisamment longs pour obtenir le lissage désiré des particules sous forme de granules ou de sphérules. Le séchage est conduit de manière à ce que la température de la biomasse ne dépasse jamais 35°C.

Ce caractère lisse et non poreux des particules de biomasse obtenues peut être caractérisé par :
- la surface spécifique des particules suivant la méthode de Brunann, Emmet et Teller ou surface BET exprimée en m² par gramme. Elle est mesurée par adsorption de krypton sur un échantillon dégazé à la température de l'azote liquide à l'aide d'un appareil ACCUSORB fabriqué par MICROMERITICS. Cette méthode repose sur la théorie d'adsorption en multi-couches d'un gaz inerte et consiste à déterminer la quantité de gaz adsorbé sur la surface en monocouche. Plus les particules seront lisses et non poreuses, plus cette surface sera petite ;
- la mesure de la porosité par immersion du matériau dans le mercure sous pression sur appareil AUTOPORE 9200 fabriqué par MICROMERITICS et détermination du volume poreux en cm³ par gramme à 17000 PSIA. Plus ces grandeurs seront petites, moins les particules seront poreuses.

Les particules obtenues après séchage en lit fluidisé à forte hauteur de couches ou en séchoir rotatif auront :
- une surface BET inférieure à 0,5 m² par gramme, de préférence à 0,2 m² par gramme, et encore de préférence à 0,1 m² par gramme.
- un volume poreux inférieur à 0,2 cm³ par gramme, de préférence inférieur à 0,1 cm3 par gramme et encore de préférence inférieur à 0,05 cm³ par gramme.

Les résultats obtenus sont les suivants :

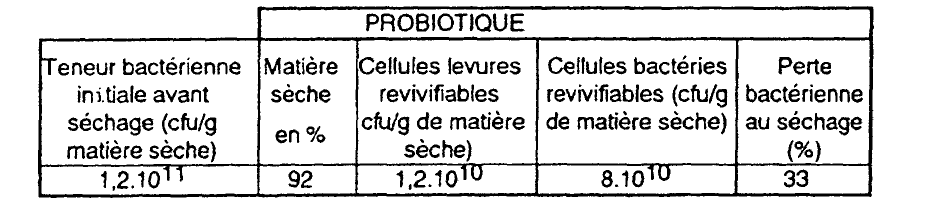

L'obtention de biomasse sèche de levure associée ou non avec des bactéries sous forme de particules lisses et non poreuses telles que définies ci-dessus est très importante pour l'utilisation comme probiotique, car plus les particules seront lisses et non poreuses, plus elles résisteront à la granulation ou pelletisation après leur incorporation dans les aliments du bétail, ceux-ci étant en général distribués au bétail sous forme de gros granulés ou pellets. Ce faible volume poreux limite le contact des granules avec la vapeur lors de la granulation ou pelletisation de l'aliment complet pour le bétail, ce qui est très important car les cellules vivantes à plus de 90 % de matière sèche résistent bien à la chaleur sèche mais non à la chaleur humide.

### EXEMPLE 6 : STABILITE DES PARTICULES LISSEES A LA CHALEUR, A LA PRESSION ET A L'HUMIDITE

On réalise selon les modalités décrites dans l'exemple précédent et avec les mêmes souches des particules sèches et lisses à au moins 93 % de matière sèche ayant les caractéristiques suivantes :

| | Levure seule | Levure + bactérie |
|---|---|---|
| Nombre cfu levures par gramme de matière sèche | 1,3.10¹⁰ | 1,2.10¹⁰ |
| Nombre cfu bactéries par gramme de matière sèche | 0 | 8.10¹⁰ |
| Matière sèche | 93 | 93 |
| Surface spécifique en m²/g des particules | 0,12 | 0,12 |
| Volume poreux en cm³/g des particules | 0,03 | 0,03 |

Les particules obtenues sont mélangées à raison de 2 ^{•}/•• dans un aliment farine. Cet aliment est ensuite granulé dans une presse ROUSSELLE type RP 3563 LSP 225 M de 37 kW à filière annulaire verticale tournante alimentée en vapeur à 2,8 bars et 145°C. La filière avait un diamètre de 5 mm et une épaisseur de 60 mm. Le temps de séjour moyen dans la presse était d'environ 13 secondes. La presse était alimentée avec le mélange conditionné à 40°C. La température des granules d'aliment du bétail ou pellets à la sortie de la filière était de 65°C.

Les gros granules ou pellets obtenus sont refroidis, et on effectue selon les méthodes classiques un dénombrement des cellules vivantes ou cfu. Bien entendu, il avait été vérifié que les particules sèches de levures ou de levures et bactéries étaient le seul apport de cellules vivantes dans la farine-aliment à granuler. De manière étonnante, alors que lors de l'ajout dans les mêmes conditions des levures instantanées de panification ou de toutes les formes connues de bactéries lactiques lyophilisées on constate une inactivation ou mortalité de pratiquement toutes les cellules de levures vivantes et de bactéries vivantes ajoutées dans l'aliment granulé, les dénombrements de cellules revivifiables de levures et de bactéries dans l'aliment granulé avec les particules sèches lisses et non poreuses de levures ou de levures plus bactéries décrites ci-dessus permettent de retrouver la quasi-totalité ou la totalité de l'apport initial de cellules vivantes dû à l'incorporation des particules sèches lisses et non poreuses de biomasse vivante à 2 ^{•}/•• de l'aliment. Le passage des granules de particules lisses et non poreuses de cellules vivantes à la température létale de 65°C pendant quelques secondes, au sein d'un aliment du bétail, à la même température, n'entraîne aucune perte appréciable de viabilité par les dénombrements classiques.

Comme de bien entendu, les exemples d'association de levures et de bactéries décrits dans les exemples ci-dessus, ne sont pas limitatifs, l'invention inclut toutes les associations sous forme de particules sèches substantiellement identiques de levures et de bactéries contenant moins de 5 % de corps étranger, de préférence à moins de 3 % de corps étranger, et encore de préférence à moins de 2 % de corps étranger. L'invention permet notamment d'obtenir des particules sèches substantiellement identiques contenant au moins 1.10¹⁰ cfu par gramme de levures oenologiques (Saccharomyces cerevisiae sous variété cerevisiae ou bayanus) et au moins 1.10¹⁰ de bactéries lactiques opérant la fermentation malo-lactique comme Lactobacillus plantarum ou de préférence Leuconostoc oenos.

## Revendications

1. Biomasse sous forme de petites particules sèches, substantiellement régulières et identiques à au moins 92% de matières sèches et de préférence au moins 93% de matières sèches contenant par gramme:
au moins 1.10⁹, de préférence au moins 3.10⁹ et encore plus préférentiellement au moins 1.10¹⁰ cellules revivifiables d'une ou plusieurs souches de bactéries lactiques
et au moins 5.10⁹, de préférence au moins 1.10¹⁰ cellules revivifiables d'une ou plusieurs souches de levures et
moins de 5% et de préférence moins de 3% de matière étrangère comprenant essentiellement des auxiliaires technologiques utilisés lors du séchage des cellules vivantes.

2. Biomasse selon la revendication 1, **caractérisée par le fait que** les souches de bactéries lactiques appartiennent à l'un des genres Lactobacillus, Streptococcus, Leuconostoc et de préférence aux espèces:
Lactobacillus brevis,
Lactobacillus plantarum,
Lactobacillus casei,
Lactobacillus delbruekii,
Lactobacillus San Francisco,
Streptococcus faecium,
Leuconostoc oenos.

3. Biomasse selon les revendications 1 ou 2, **caractérisée par le fait que** les souches de levures appar tiennent à la famille des Saccharomycetaceae, de préférence au genre Saccharomyces.

4. Biomasse selon l'une des revendications 1 à 3, **caractérisée par le fait qu'**elle comprend, d'une part, des souches de bactéries du genre Lactobacillus, associant des souches homofermentaires et hétérofermentaires et, d'autre part, une souche de levure appartenant au genre Saccharomyces qui ne fermente pas le maltose telle que la souche Saccharomyces cerevisiae variété chevalieri.

5. Biomasse selon l'une des revendications 1 à 3, **caractérisée par le fait qu'**elle comprend une souche de bactéries appartenant à l'espèce Streptococcus faecium et une souche de levure appartenant à l'espèce Saccharomyces cerevisiae, ces souches ayant été sélectionnées en vue de l'utilisation de la biomasse comme probiotique en alimentation animale.

6. Biomasse selon l'une des revendications 1 à 3, **caractérisée par le fait qu'**elle comprend une souche de bactéries appartenant soit au genre Lactobacillus, soit à l'espèce Leuconostoc oenos sélectionnée pour sa propriété d'opérer la fermentation malolactique dans les moûts ou les vins et, comme souche de levures, une souche de levure oenologique.

7. Biomasse selon l'une des revendications 1 à 6, **caractérisée par le fait que** ses particules constitutives se présentent sous la forme de granules ou de sphérules secs homogènes dans lesquels les cellules de levures et de bactéries sont intimement mélangées.

8. Biomasse selon l'une des revendications 1 à 6, **caractérisée par le fait que** ses particules constitutives substantiellement identiques sont des granules constitués de cellules de levures et enrobés à leur périphérie par les cellules de bactéries lactiques.

9. Procédé de préparation d'une biomasse sous forme de petites particules sèches régulières selon l'une des revendications 1 à 8, **caractérisé par le fait que** l'on utilise les cellules de levure comme agent auxiliaire technologique de protection des bactéries lactiques lors de la dessiccation.

10. Procédé de préparation selon la revendication 9 d'une biomasse selon l'une des revendications 1 à 7, **caractérisé par le fait:**
- **qu'**on mélange une crème de bactéries avec de la levure pressée ayant des matières sèches comprises entre environ 30 et 33%,
- **qu'**on ajoute éventuellement à ce mélange pâteux des auxiliaires technologiques protégeant la biomasse lors du séchage,
- **qu'**on extrude cette biomasse en vermicelles de diamètre inférfieur à 3 mm et de préférence inférieur à 1 mm,
- **qu'**on sèche les vermicelles obtenus dans un courant d'air chaud comme le séchage par fluidisation jusqu'à au moins 92% de matières sèches, de préférence au moins 93% de matières sèches.

11. Procédé de préparation selon la revendication 9 d'une biomasse selon l'une des revendications 1 à 6 et 8, **caractérisé par le fait:**
- **qu'**on sèche séparément la levure de manière à obtenir des fins granules de diamètre inférieur à 1 mm,
- **qu'**on pulvérise sur lesdits granules en mouvement une crème de bactéries lactiques à au moins 14% de matières sèches contenant éventuellement des auxiliaires technologiques ou des levures les protégeant lors de la dessiccation,
- **qu'**on effectue éventuellement un séchage complémentaire dans un courant d'air chaud comme un séchage par fluidisation de manière à amener les granules à au moins 92% de matières sèches et de préférence à au moins 93% de matières sèches.

12. Procédé selon la revendication 11, **caractérisé par le fait que** la levure est séchée à au moins 95% de matières sèches en présence d'un agent auxiliaire technologique de séchage du genre de ceux qui sont utilisés lors de la fabrication des levures sèches instantanées.

13. Procédé selon l'une des revendications 10 à 12, **caractérisé par le fait que** l'on a recours à un ou plusieurs agents auxiliaires technologiques choisis dans le groupe comprenant le monostéarate de sorbitan, la carboxyméthylcellulose et le glycérol.

14. Procédé selon la revendication 10, **caractérisé par le fait que** la biomasse obtenue est séchée dans un fluidiseur à forte hauteur de couche ou dans un séchoir rotatif de manière à obtenir des particules lisses et non poreuses.

15. Biomasse selon l'une des revendications 1 à 3, 5 et 7, constituant un probiotique dans lequel les cellules vivantes de levures et de bactéries se présentent sous la forme de particules lisses et non poreuses ayant les caractéristiques suivantes:
- surface spécifique BET inférieure à 0,5 m² par gramme, de préférence inférieure à 0,2 m² par gramme et encore de préférence inférieure à 0,1 m² par gramme,
- porosité inférieure à 0,2 cm³/g, de préférence inférieure à 0,1 cm³/g et encore de préférence inférieure à 0,05 cm³/g,
ledit probiotique, en mélange avec des aliments du bétail, pouvant être porté, lors de la granulation de l'aliment du bétail, à 65°C pendant quelques secondes sans perte de viabilité.

16. Utilisation comme ferment panaire d'une biomasse selon l'une des revendications 1 à 4 ou 7 et 8.

17. Utilisation comme ferment vinique d'une biomasse selon l'une des revendications 1 à 3 ou 6 à 8.

## Patentansprüche

1. Biomasse in Form von kleinen, im Wesentlichen gleichförmigen und identischen trockenen Partikeln, mit mindestens 92% Trockenmasse und bevorzugt mindestens 93% Trockenmasse, enthaltend pro Gramm:
mindestens 1•10⁹, bevorzugt mindestens 3•10⁹ und stärker bevorzugt mindestens 1•10¹⁰ wiederbelebbare Zellen eines oder mehrerer Milchbakterienstämme,
und mindestens 5•10⁹, bevorzugt mindestens 1•10¹⁰ wiederbelebbare Zellen eines oder mehrerer Hefestämme, und
weniger als 5%, bevorzugt weniger als 3% Fremdmasse, umfassend im Wesentlichen die bei der Trocknung der lebenden Zellen verwendeten technischen Hilfsmittel.

2. Biomasse nach Anspruch 1, **dadurch gekennzeichnet, dass** die Milchbakterien einer der Gattungen Lactobacillus, Streptococcus, Leuconostoc angehören, und bevorzugt einer der Spezies:
Lactobacillus brevis,
Lactobacillus plantarum,
Lactobacillus casei,
Lactobacillus delbruekii,
Lactobacillus San Francisco,
Streptococcus faecium,
Leuconostoc oenos.

3. Biomasse nach Anspruch 1 oder 2, **dadurch gekennzeichnet, dass** die Hefestämme der Familie der Saccharomycetaceae, bevorzugt der Gattung Saccharomyces angehören.

4. Biomasse nach einem der Ansprüche 1 bis 3, **dadurch gekennzeichnet, dass** sie einerseits Bakterienstämme der Gattung Lactobacillus, sowohl homofermentierende als auch heterofermentierende Stämme, und andererseits einen der Gattung Saccharomyces angehörenden Hefestamm, der Maltose nicht fermentiert, wie etwa den Stamm Saccharomyces cerevisiae Varietät chevalieri, umfasst.

5. Biomasse nach einem der Ansprüche 1 bis 3, **dadurch gekennzeichnet, dass** sie einen der Spezies Streptococcus faecium angehörenden Bakterienstamm und einen der Gattung Saccharomyces cerevisiae angehörenden Hefestamm umfasst, wobei diese Stämme im Hinblick auf eine Verwendung der Biomasse als Probiotikum bei der Ernährung von Tieren ausgewählt worden sind.

6. Biomasse nach einem der Ansprüche 1 bis 3, **dadurch gekennzeichnet, dass** sie einen Bakterienstamm, welcher entweder der Gattung Lactobacillus oder der Spezies Leuconostoc oenos angehört, ausgewählt im Hinblick auf seine Eigenschaft den biologischen Säureabbau in Mosten oder Weinen anzutreiben, und als Hefestamm einen Weinhefestamm umfasst.

7. Biomasse nach einem der Ansprüche 1 bis 6, **dadurch gekennzeichnet, dass** die Partikel, aus denen sie gebildet ist, in Form von trockenen homogenen Körnchen oder Kügelchen vorliegen, in denen die Hefe- und Bakterienzellen innig miteinander vermischt sind.

8. Biomasse nach einem der Ansprüche 1 bis 6, **dadurch gekennzeichnet, dass** die im Wesentlichen identischen Partikel, aus denen sie gebildet ist, Körnchen sind, die aus Hefezellen gebildet sind und auf ihrem Äußeren von Milchbakterienzellen umhüllt sind.

9. Verfahren zur Herstellung einer Biomasse in Form von kleinen regelmäßigen trockenen Partikeln nach einem der Ansprüche 1 bis 8, **dadurch gekennzeichnet, dass** man die Hefezellen als technisches Hilfsmittel zum Schutz der Milchbakterien bei der Trocknung verwendet.

10. Verfahren nach Anspruch 9 zur Herstellung einer Biomasse nach einem der Ansprüche 1 bis 7, **dadurch gekennzeichnet dass** man:
- eine Bakteriencreme mit Presshefe mit einer Trockenmasse zwischen etwa 30 und 33% vermischt,
- zu diesem pastösen Gemisch gegebenenfalls technische Hilfsmittel zugibt, welche die Biomasse bei der Trocknung schützen,
- diese Biomasse zu Strängen mit einem Durchmesser von kleiner als 3 mm und bevorzugt von kleiner als 1 mm extrudiert,
- die erhaltenen Stränge in einem warmen Luftstrom trocknet, wie etwa durch Fliessbetttrocknen, bis zu einer Trockenmasse von mindestens 92%, bevorzugt einer Trockenmasse von mindestens 93%.

11. Verfahren nach Anspruch 9 zur Herstellung einer Biomasse nach einem der Ansprüche 1 bis 6 und 8, **dadurch gekennzeichnet dass** man:
- die Hefe separat trocknet, derart so dass man feine Körnchen mit einem Durchmesser kleiner als 1 mm erhält,
- auf diese Körnchen, während sie bewegt werden, eine Creme von Milchbakterien mit mindestens 14% Trockenmasse, welche gegebenenfalls technische Hilfsmittel oder Hefen, die sie bei der Trocknung schützen, enthält, aufsprüht,
- gegebenenfalls eine ergänzende Trocknung in einem warmen Luftstrom, wie etwa einer Fliessbetttrocknung derart ausführt, um die Körnchen auf eine Trockenmasse von mindestens 92%, bevorzugt eine Trockenmasse von mindestens 93% zu bringen.

12. Verfahren nach Anspruch 11, **dadurch gekennzeichnet, dass** die Hefe auf eine Trockenmasse von mindestens 95% getrocknet wird, in Gegenwart eines technischen Hilfsmittels zur Trocknung von der Art, wie bei der Herstellung von Instanttrockenhefen verwendet.

13. Verfahren nach einem der Ansprüche 10 bis 12, **dadurch gekennzeichnet, dass** man eines oder mehrere technische Hilfsmittel verwendet, ausgewählt aus der Gruppe, umfassend Sorbitanmonostearat, Carboxymethylcellulose und Glyzerol.

14. Verfahren nach Anspruch 10, **dadurch gekennzeichnet, dass** die erhaltene Biomasse in einem Fließbetttrockner mit einer hohen Schichtdicke des Fliessbetts oder in einem Rotationstrockner derart getrocknet wird, so dass man glatte und nicht-poröse Partikel erhält.

15. Biomasse nach einem der Ansprüche 1 bis 3, 5 und 7, welches ein Probiotikum darstellt, wobei die lebenden Hefe- und Bakterienzellen in Form von glatten und nicht-porösen Partikeln vorliegen, mit den nachfolgenden Eigenschaften:
- einer spezifischen BET-Oberfläche von weniger als 0,5 m² pro Gramm, bevorzugt von weniger als 0,2 m² pro Gramm und stärker bevorzugt von weniger als 0,1 m² pro Gramm,
- einer Porosität von weniger als 0,2 cm³/g, bevorzugt von weniger als 0,1 cm³/g und stärker bevorzugt von weniger als 0,05 cm³/g,
wobei das Probiotikum im Gemisch mit Viehfutter bei der Granulierung des Viehfutters während mehrerer Sekunden auf 65°C gebracht werden kann ohne die Lebensfähigkeit zu verlieren.

16. Verwendung einer Biomasse nach einem der Ansprüche 1 bis 4 oder 7 und 8 als Brotferment.

17. Verwendung einer Biomasse nach einem der Ansprüche 1 bis 3 oder 6 bis 8 als Weinferment.

## Claims

1. Biomass in the form of small dry particles, substantially regular and identical, and with at least 92% dry matter and preferably at least 93% dry matter, containing per gram:
at least 1.10⁹, preferably at least 3.10⁹ and even more preferably at least 1.10¹⁰ revitalizable cells of one or more strains of lactic bacteria
and at least 5.10⁹, preferably at least 1.10¹⁰ revitalizable cells of one or more strains of yeasts
less than 5% and preferably less than 3% of extraneous material comprising essentially technological auxiliary substances used during the drying of the living cells.

2. Biomass according to Claim 1, **characterized by** the fact that the strains of lactic bacteria belong to one of the genera Lactobacillus, Streptococcus, Leuconostoc and preferably to the species:
Lactobacillus brevis,
Lactobacillus plantarum,
Lactobacillus casei,
Lactobacillus delbruekii,
Lactobacillus San Francisco,
Streptococcus faecium,
Leuconostoc oenos.

3. Biomass according to Claims 1 or 2, **characterized by** the fact that the strains of yeast belong to the family of the Saccharomycetaceae, and preferably to the genus Saccharomyces.

4. Biomass according to one of the Claims 1 to 3, **characterized by** the fact that it comprises firstly strains of bacteria of the genus Lactobacillus, combining homofermentative and heterofermentative strains, and secondly a strain of yeast belonging to the Saccharomyces genus that does not ferment maltose, such as the strain Saccharomyces cerevisiae variety chevalieri.

5. Biomass according to one of the Claims 1 to 3, **characterized by** the fact that it comprises a strain of bacteria belonging to the species Streptococcus faecium and a strain of yeast belonging to the species Saccharomyces cerevisiae, these strains having been chosen with the intention of using the biomass as a probiotic in animal nutrition.

6. Biomass according to one of the Claims 1 to 3, **characterized by** the fact that it comprises a strain of bacteria belonging either to the genus Lactobacillus or to the species Leuconostoc oenos, chosen for its property of performing the malo-lactic fermentation in musts/worts or wines, and as the yeast strain an oenological (wine-making) strain of yeast.

7. Biomass according to one of the Claims 1 to 6, **characterized by** the fact that its constituent particles are in the form of homogeneous dry granules or spherules in which the cells of yeast and bacteria are intimately mixed.

8. Biomass according to one of the Claims 1 to 6, **characterized by** the fact that its substantially identical constituent particles are granules consisting of yeast cells and coated at their periphery with cells of lactic bacteria.

9. Process for preparing a biomass in the form of small regular dry particles according to one of the Claims 1 to 8, **characterized by** the fact that yeast cells are used as the auxiliary technological agent to protect the lactic bacteria during the desiccation (drying) process.

10. Process of preparation according to Claim 9 of a biomass according to one of the Claims 1 to 7, **characterized by** the fact:
- that a cream of bacteria is mixed with pressed yeast having dry matter comprised between about 30 and 33%,
- that technological auxiliaries protecting the biomass during drying are optionally added to this pasty mixture,
- that this biomass is extruded as vermicelli (thin noodles) with a diameter smaller than 3 mm and preferably smaller than 1 mm,
- that the vermicelli obtained are dried in a current of hot air such as fluidisation drying down to at least 92% dry matter and preferably at least 93% dry matter.

11. Process of preparation according to Claim 9 of a biomass according to one of the Claims 1 to 6 and 8, **characterized by** the fact:
- that the yeast is dried separately in such a way as to obtain fine granules with a diameter smaller than 1 mm,
- that a cream of lactic bacteria with at least 14% of dry matter and optionally containing technological auxiliaries or yeasts protecting them during the desiccation is sprayed onto the said granules in movement,
- that optionally a complementary drying in a current of hot air such as fluidisation drying is performed in such a way as to bring the granules to at least 92% dry matter and preferably to at least 93% dry matter.

12. Process according to Claim 11, **characterized by** the fact that the yeast is dried to at least 95% dry matter in the presence of an auxiliary technological drying agent of the type used during the manufacture of instant dried yeasts.

13. Process according to one of the Claims 10 to 12, **characterized by** the fact that use is made of one or more auxiliary technological agents chosen from among the group comprising sorbitan monostearate, carboxymethylcellulose and glycerol.

14. Process according to Claim 10, **characterized by** the fact that the biomass obtained is dried in a deep-bed fluidiser or in a rotary drier in such a way as to obtain particles that are smooth and non-porous.

15. Biomass according to one of the Claims 1 to 3, 5 and 7, constituting a probiotic in which the living cells of yeasts and bacteria are present in the form of smooth, non-porous particles having the following characteristics:
- BET specific surface area less than 0.5 m² per gram, preferably less than 0.2 m² per gram and even more preferably less than 0.1 m² per gram,
- porosity less than 0.2 cm³/g, preferably less than 0.1 cm³/g and even more preferably less than 0.05 cm³/g,
the said probiotic, when mixed with livestock fodder, having the ability to be taken, during the granulation of the livestock fodder, to 65°C for a few seconds without losing viability.

16. Use of a biomass according to one of the Claims 1 to 4 or 7 and 8 as a bread-making leaven (ferment).

17. Use of a biomass according to one of the Claims 1 to 3 or 6 to 8 as a wine-making ferment.
